# EUROPEAN PATENT APPLICATION

(11) **EP 4 755 286 A1**
(43) Date of publication of application: **10.06.2026**
(21) Application number: 24865167.1
(22) Date of filing: 19.08.2024
(51) Int. Cl.: A61B 1/015, A61B 1/00, A61B 1/012, A61B 1/12

(54) **CONNECTOR FOR AUXILIARY WATER FEED PORT SLEEVE OF ENDOSCOPE**

(30) Priority: 15.09.2023 JP 2023150176
(71) Applicant: Ort Medical Co., Ltd., Yokohama-shi, Kanagawa, 222-0033 (JP)
(72) Inventor: KARASAWA Koji, Yokohama-shi, Kanagawa 222-0033 (JP)
(74) Representative: Hafner & Kohl PartmbB
(86) International application number: PCT/JP2024/029253
(87) International publication number: WO 2025/057661

(57) **Abstract**

There is provided a low-cost system that allows a water-supply tube unit to be connected to an auxiliary water feed port of an endoscope. A connector 80 for an auxiliary water feed port of the present invention has a tubular connector main body 80a made of a synthetic resin. The connector main body 80a has: an insertion part 81 that is inserted into a housing space 42b of an auxiliary water feed port sleeve 42; an external thread 83 that engages with an internal thread 42c in the housing space 42b of the auxiliary water feed port sleeve 42; and a step 87 that is provided between the insertion part 81 and the external thread 83 in a central axis direction of the connector main body 80a, along an entire circumference of an outer circumferential surface of the connector main body 80a. The auxiliary water feed port sleeve 42 has a tapered surface 42f between the housing space 42b and the internal thread 42c in a central axis direction of the auxiliary water feed port sleeve 42, the tapered surface 42f being reduced in diameter from the internal thread 42c toward the housing space 42b. A ridge of the step 87 provided along the entire circumference of the outer circumferential surface of the connector main body 80a is held in pressure-contact with the tapered surface 42f.

## Description

### Technical Field

The present invention relates to a connector for an auxiliary water feed port sleeve of an endoscope, the connector being configured to fasten and fix a pump tube unit when the pump tube unit is attached to the connector for an auxiliary water feed port sleeve.

### Background Art

In the medical field, examinations and surgeries using an endoscope have become common. An endoscope is equipped with an opening called an auxiliary water feed port in the distal end face of an insertion part to be inserted into the body cavity of a patient. The auxiliary water feed port is provided to wash a target site inside the body cavity of a patient concurrently with the use of a treatment tool using a forceps channel. The auxiliary water feed port is coupled to an auxiliary water feed port sleeve by a dedicated channel provided inside the endoscope. When using the endoscope, a water-supply tube unit is attached to the auxiliary water feed port sleeve through a check valve unit (e.g., see Patent Literature 1 and Patent Literature 2). Thus, a liquid supplied into the endoscope through the water-supply tube unit is ejected from the auxiliary water feed port through the aforementioned channel.

### Citation List

### Patent Literature

Patent Literature 1: Japanese Patent Laid-Open No. 8-238214
Patent Literature 2: Japanese Patent Laid-Open No. 2005-160772

### Summary of Invention

### Technical Problem

As part of infection control, the endoscope is washed after each use. The check valve unit is provided as a dedicated unit to be attached to the auxiliary water feed port of the endoscope. After the use of the endoscope, the check valve unit is removed from the endoscope and washed separately from the endoscope, and is then attached to the auxiliary water feed port of the endoscope when the endoscope is used. Therefore, as the number of times the check valve unit is attached to and detached from the auxiliary water feed port of the endoscope increases, the liquid tightness between the check valve unit and the check valve sleeve decreases, potentially leading to leakage.

The check valve units disclosed in Patent Literature 1 and Patent Literature 2 have configurations in which a rubber plug and an O-ring are attached to a check valve sleeve made of a metal material, such as stainless steel. While such a check valve unit has an advantage that it can be repeatedly used by washing it, it has the drawback that the manufacturing cost is high compared with check valves used for other purposes. Moreover, this check valve unit needs to be washed after each use and thus requires time and effort for washing. As such, there is a strong demand for a low-cost system that allows a water-supply tube unit to be connected to an auxiliary water feed port of an endoscope without using a check valve unit.

The present invention has been developed to solve the above-described problem, and an objective thereof is to provide an inexpensive system that allows a water-supply tube unit to be connected to an auxiliary water feed port of an endoscope.

### Solution to Problem

A connector for an auxiliary water feed port sleeve of an endoscope in one aspect of the present invention is a connector for an auxiliary water feed port sleeve used to connect a tube unit installed in a water supply device to an auxiliary water feed port sleeve of an endoscope, characterized in that: the connector has a tubular connector main body made of a synthetic resin; the connector main body has an insertion part that is inserted into a housing space provided in the auxiliary water feed port sleeve, an external thread that is provided on an outer circumferential surface of the connector main body and engages with an internal thread provided in the housing space of the auxiliary water feed port sleeve, and a step that is provided between the insertion part and the external thread in a central axis direction of the connector main body, along an entire circumference of the outer circumferential surface of the connector main body; the auxiliary water feed port sleeve has a tapered surface between the housing space and the internal thread in a central axis direction of the auxiliary water feed port sleeve, the tapered surface being reduced in diameter from the internal thread toward the housing space; and when the connector main body is fastened and fixed to the auxiliary water feed port sleeve, a ridge of the step provided along the entire circumference of the outer circumferential surface of the connector main body is held in pressure-contact with the tapered surface.

It is preferable that the connector main body include a first flow passage which is provided on one end side in the central axis direction of the connector main body and into which a liquid from the tube unit flows, and a second flow passage which is provided on the other end side in the central axis direction of the connector main body and from which the liquid flowing into the first flow passage is discharged into the housing space; that an inside diameter of the second flow passage be smaller than an inside diameter of the first flow passage; and that the inside diameter of the first flow passage be smaller than an inside diameter of the housing space.

It is also preferable that the housing space include, at a bottom part of the housing space, a water supply port that communicates with an auxiliary water supply channel of the endoscope; that an inside diameter of the water supply port be smaller than the inside diameter of the second flow passage; and that an outside diameter of the insertion part be equal to the inside diameter of the housing space.

It is preferable to connect a check valve, which is fixed at one end of the tube unit, to one end of the connector main body along its central axis, on the side opposite to the end connected to the auxiliary water feed port sleeve.

### Advantageous Effect of Invention

The present disclosure can provide a low-cost system that enables a water-supply tube unit to be connected to an auxiliary water feed port of an endoscope.

### Brief Description of Drawings

[Fig. 1] Fig. 1 is a schematic view showing one configuration of an endoscope system in an embodiment.
[Fig. 2] Fig. 2 is a schematic view showing one configuration of a nozzle tip provided in an endoscope insertion part.
[Fig. 3] Fig. 3 is a schematic view showing, in an exploded view, the configurations of an auxiliary water feed port sleeve provided at a connection part of the endoscope, a connector for an auxiliary water feed port sleeve, and a check valve.
[Fig. 4] Fig. 4 is a schematic sectional view showing the configuration of the auxiliary water feed port sleeve.
[Fig. 5] Fig. 5 is a schematic view showing the configuration of the check valve, partially in cross-section.
[Fig. 6] Fig. 6 is a schematic sectional view showing the configuration of the connector for an auxiliary water feed port sleeve.
[Fig. 7] Fig. 7 is a schematic view showing the procedure for attaching the connector for an auxiliary water feed port sleeve and the check valve to the auxiliary water feed port sleeve provided at the connection part of the endoscope.
[Fig. 8] Fig. 8 is a schematic view showing a region A indicated in Fig. 7(c) in close-up.
[Fig. 9] Fig. 9 is a table showing a measurement result of the discharge amount of liquid discharged from an auxiliary water feed port 36 when a pump tube unit A attached to a water supply device 50A was connected to an endoscope 20.
[Fig. 10] Fig. 10 is a table showing a measurement result of the discharge amount of a liquid discharged from the auxiliary water feed port 36 when a pump tube unit B attached to the water supply device 50A was connected to the endoscope 20.
[Fig. 11] Fig. 11 is a table showing a measurement result of the discharge amount of a liquid discharged from the auxiliary water feed port 36 when the pump tube unit B attached to a water supply device 50B was connected to the endoscope 20.
[Fig. 12] Fig. 12 is a table summing up the presence or absence of liquid leakage and the pressure of a discharged liquid when the pump tube unit B attached to the water supply device 50B was connected to the endoscope 20.
[Fig. 13] Fig. 13 is a table summing up the presence or absence of liquid leakage and the pressure of a discharged liquid when the pump tube unit A attached to the water supply device 50A was connected to the endoscope 20.
[Fig. 14] Fig. 14(a) and Fig. 14(b) are tables summing up a discharge amount and a water retention time, respectively, when the pump tube unit B attached to the water supply device 50B was connected to the endoscope 20.
[Fig. 15] Fig. 15(a) and Fig. 15(b) are tables summing up a discharge amount and a water retention time, respectively, when a pump tube unit C attached to the water supply device 50B was connected to the endoscope 20.
[Fig. 16] Fig. 16(a) and Fig. 16(b) are tables summing up a discharge amount and a water retention time, respectively, when the pump tube unit B attached to the water supply device 50A was connected to the endoscope 20.
[Fig. 17] Fig. 17(a) and Fig. 17(b) are tables summing up a discharge amount and a water retention time, respectively, when the pump tube unit C attached to the water supply device 50A was connected to the endoscope 20.
[Fig. 18] Fig. 18(a) and Fig. 18(b) are tables summing up a discharge amount and a water retention time, respectively, when the pump tube unit A attached to the water supply device 50A was connected to the endoscope 20.

### Description of Embodiments

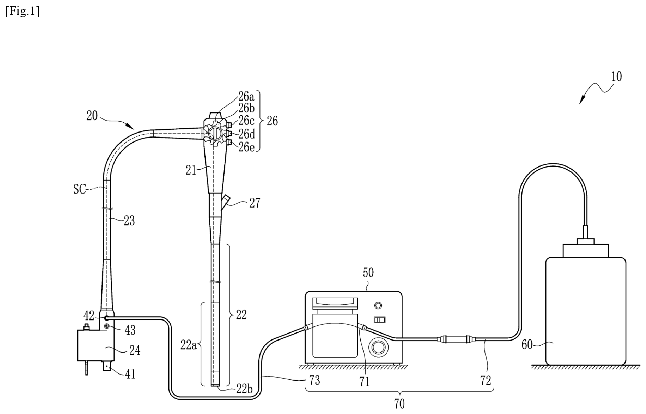

In the following, an endoscope system that is one embodiment of the present invention will be described with reference to the drawings. As shown in Fig. 1, an endoscope system 10 includes an endoscope 20, a water supply device 50, a water supply tank 60, a pump tube unit 70, and others.

The endoscope 20 has a manipulation handle 21, an endoscope insertion part 22, a universal tube 23, and a connection part 24. The manipulation handle 21 includes a plurality of manipulation parts 26, a forceps insertion part 27, and others. The manipulation parts 26 has, for example, other than angle knobs 26a, 26b, various buttons such as an air/water supply button 26c, a suction button 26d, and a shutter button 26e.

The angle knobs 26a, 26b are rotationally moved when performing a bending motion of a bending portion 22a provided on a distal end side of the endoscope insertion part 22. The angle knob 26a is rotationally manipulated when bending the bending portion 22a of the endoscope insertion part 22 in, for example, a direction orthogonal to the plane of the sheet of Fig. 1. The angle knob 26b is rotationally manipulated when bending the bending portion 22a of the endoscope insertion part 22 in, for example, a left-right direction in the plane of the sheet of Fig. 1.

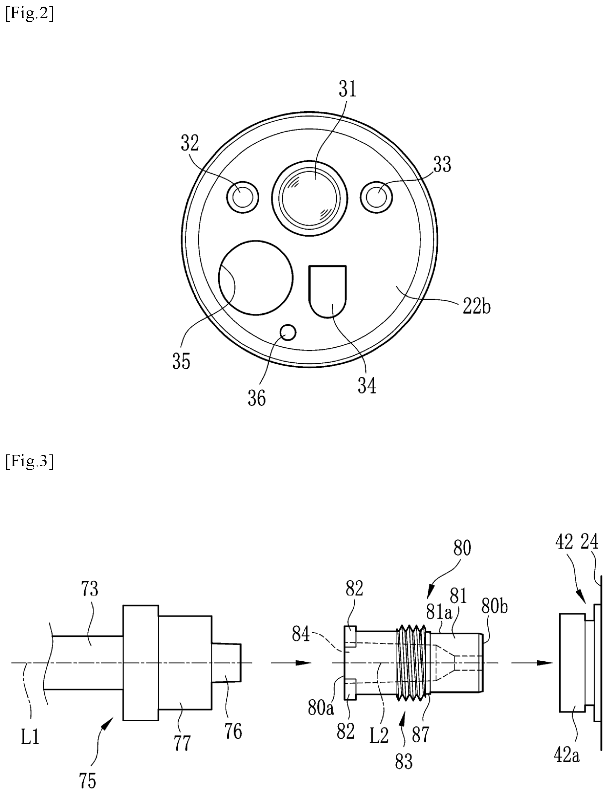

The air/water supply button 26c is manipulated when washing an observation port 31 and illumination ports 32, 33 (see Fig. 2) provided in a distal end face 22b of the endoscope insertion part 22. The suction button 26d is manipulated when suctioning waste matter in a body cavity (including a liquid used to wash a target site inside a body cavity) from a forceps port 35 provided at the distal end of the endoscope insertion part 22. The shutter button 26e is manipulated when imaging the inside of a body cavity using an imaging device (not shown) provided inside the distal end of the endoscope insertion part 22.

A treatment tool, such as forceps, is inserted into the forceps insertion part 27. A forceps plug is attached to the forceps insertion part 27 when the endoscope 20 is not used.

While this is not illustrated in detail, inside the endoscope insertion part 22, an air supply channel, a water supply channel, a light guide, a forceps channel, and an auxiliary water supply channel are provided. Hereinafter, the auxiliary water supply channel may be denoted by a reference character SC when described.

The light guide guides light emitted from a light source device (not shown), to which the connection part 24 is connected, to the illumination ports 32, 33 (see Fig. 2) provided at the distal end of the endoscope insertion part 22. The forceps channel guides the treatment tool, such as forceps, inserted from the forceps insertion part 27 provided in the manipulation handle 21 to the forceps port 35 (see Fig. 2) provided at the distal end of the endoscope insertion part 22. The auxiliary water supply channel SC guides a liquid, such as saline, to an auxiliary water feed port 36 (see Fig. 2) provided at the distal end of the endoscope insertion part 22. The liquid, such as saline, is supplied to the auxiliary water supply channel SC as the water supply device 50 is driven and then discharged from the aforementioned auxiliary water feed port 36.

As shown in Fig. 2, in the distal end face 22b of the endoscope insertion part 22, the observation port 31, the illumination ports 32, 33, an air/water supply nozzle 34, the forceps port 35, the auxiliary water feed port 36, and others are provided. The configuration of the distal end face 22b of the endoscope insertion part 22 shown in Fig. 2 merely represents one example, and the types and positions of the openings provided on the distal end face 22b of the endoscope insertion part 22 can be set as appropriate.

The observation port 31 exposes an objective lens of the imaging device provided inside the distal end of the endoscope insertion part 22. The objective lens takes an observed image of a target site inside a body cavity into the imaging device. The illumination ports 32, 33 emit light having been guided by the light guide as illumination light toward the target site inside the body cavity and the vicinity of the target site. The air/water supply nozzle 34 ejects air or a liquid toward the observation port 31 and the illumination ports 32, 33 to wash the observation port 31 and the illumination ports 32, 33 as well as their vicinity. The forceps port 35 allows the treatment tool, such as forceps, inserted from the forceps insertion part 27 to be taken in and out from the distal end face 22b. As the water supply device 50 is driven, the auxiliary water feed port 36 ejects a liquid to be supplied to the auxiliary water supply channel SC of the endoscope 20 through the pump tube unit 70.

Returning to Fig. 1, description will be continued. The universal tube 23 connects the manipulation handle 21 and the connection part 24 to each other. While this is not illustrated, inside the universal tube 23, the light guide, the suction channel, and others are provided other than the auxiliary water supply channel SC.

The connection part 24 includes a light source connection part 41 that allows connection to the light source device (not illustrated), an auxiliary water feed port sleeve 42 to which one end of the pump tube unit 70 is connected, a suction port 43, and others. As shown in Fig. 3, a check valve 75 fixed at one end of the pump tube unit 70 is attached to the auxiliary water feed port sleeve 42 through a connector 80 for an auxiliary water feed port sleeve.

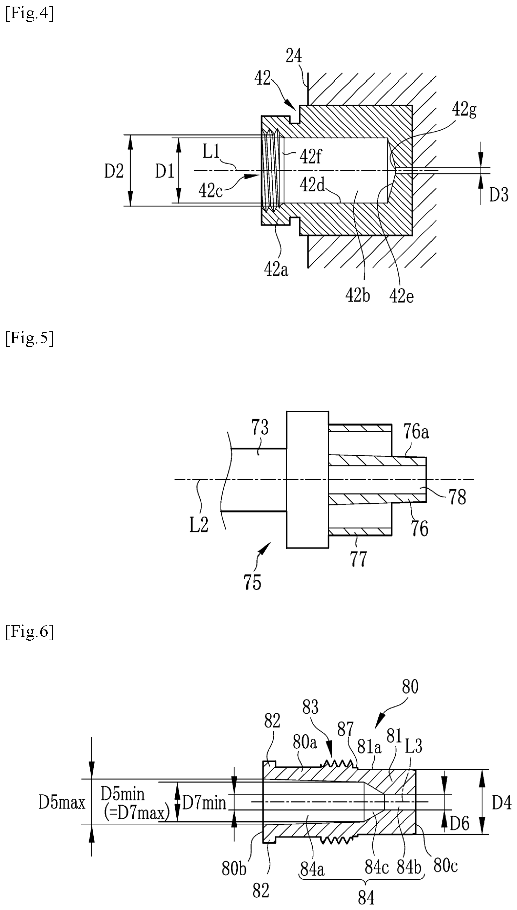

As shown in Fig. 4, the auxiliary water feed port sleeve 42 is provided at the connection part 24, with a head portion 42a of the auxiliary water feed port sleeve 42 protruding from the connection part 24. The auxiliary water feed port sleeve 42 has a housing space 42b extending from the head portion 42a in the direction of a central axis (L1) of the auxiliary water feed port sleeve 42.

In the housing space 42b, an internal thread 42c is provided at an end portion on the side of the head portion 42a in the direction of the central axis (L1). An external thread 83 of the connector 80 for an auxiliary water feed port sleeve, to be described later, engages with the internal thread 42c. Between an inner circumferential surface 42d of the housing space 42b and the internal thread 42c, a tapered surface 42f that decreases in diameter toward a bottom part 42e of the housing space 42b is formed due to the difference in inside diameter between an inside diameter D1 of the housing space 42b and an inside diameter D2 of the internal thread 42c (D2 > D1). At the bottom part 42e of the housing space 42b, a water supply port 42g that communicates with the auxiliary water supply channel SC is formed.

Returning to Fig. 1, description will be continued. The water supply device 50 is a device that supplies a liquid stored in the water supply tank 60 toward the endoscope 20. While this is not illustrated, the water supply device 50 is a device that has, for example, a plurality of rollers and a retention member for retaining these rollers at regular angular intervals, and that repeatedly performs an action of discharging the liquid while drawing in a new liquid by squeezing a pump tube 71 by one of the rollers as the retention member rotates. The water supply device 50 is driven by, for example, an operation of pressing a foot switch (not shown). The water supply amount of the liquid that is supplied toward the endoscope 20 through the pump tube unit 70 when the water supply device 50 is driven is set to, for example, 240 ml/min.

The pump tube unit 70 includes the pump tube 71, a filter-equipped water supply tube 72, and a water supply tube 73, and has a configuration in which the filter-equipped water supply tube 72 is connected at one end in the extension direction of the pump tube 71 and the water supply tube 73 is connected at the other end in the extension direction. The filter-equipped water supply tube 72 is mounted to the water supply tank 60 at the other end, in an extension direction of the filter-equipped water supply tube 72, that is opposite from one end connected to the pump tube 71. The water supply tube 73 is mounted to the auxiliary water feed port sleeve 42 of the endoscope 20 at the other end, in an extension direction of the water supply tube 73, that is opposite from one end connected to the pump tube 71.

The above-described pump tube 71 is mounted to the water supply device 50, and by being repeatedly squeezed while the water supply device 50 is driven, draws the liquid stored in the water supply tank 60 into the pump tube 71 through the filter-equipped water supply tube 72, and at the same time discharges the liquid drawn into the pump tube 71 to the water supply tube 73.

Here, the inside diameter and the outside diameter of the pump tube 71 used for the pump tube unit 70 are, for example, 3.6 mm and 6.9 mm, respectively. The inside diameter and the outside diameter of the filter-equipped water supply tube 72 and the water supply tube 73 are, for example, 3.4 mm and 6.0 mm, respectively.

The water supply tube 73 includes the check valve 75 at one end to which the above-described auxiliary water feed port sleeve 42 is attached. For example, the check valve 75 permits liquid to flow from the pump tube unit 70 to the auxiliary water supply channel SC of the endoscope 20 while preventing liquid from flowing (backflow) from the auxiliary water supply channel SC of the endoscope 20 to the pump tube unit 70.

As shown in Fig. 5, the check valve 75 has a luer tip 76 and a luer lock part 77 at one end portion in the direction of a central axis (L2) of the check valve 75. When attaching the check valve 75 to the connector 80 for an auxiliary water feed port sleeve, the luer tip 76 is inserted into an internal space 84 provided in the connector 80 for an auxiliary water feed port sleeve. At the luer tip 76, a flow passage 78 is provided that extends in the direction of the central axis (L2) of the check valve 75 and communicates with the water supply tube 73.

The luer lock part 77 has an internal thread (not illustrated) on an inner circumferential surface of the luer lock part 77. When fastening and fixing the check valve 75 to the connector 80 for an auxiliary water feed port sleeve, the internal thread provided in the inner circumferential surface of the luer lock part 77 engages with an external thread 82 of the connector 80 for an auxiliary water feed port sleeve.

While in this embodiment the check valve 75 is fastened and fixed to the connector 80 for an auxiliary water feed port sleeve, the check valve 75 and the connector 80 for an auxiliary water feed port sleeve may alternatively be integrally formed.

The connector 80 for an auxiliary water feed port sleeve includes a connector main body 80a manufactured by molding using a synthetic resin material, such as polypropylene, nylon, fluorine resin, ABS resin, or polycarbonate. Hereinafter, the connector 80 for an auxiliary water feed port sleeve may be referred to as a connector when described.

As shown in Fig. 6, in the direction of a central axis (L3) of the connector main body 80a, the connector main body 80a has an insertion part 81 on one end side and the external thread 82 at the other end portion. When fastening and fixing the connector 80 to the auxiliary water feed port sleeve 42, the insertion part 81 is inserted into the housing space 42b of the auxiliary water feed port sleeve 42. Therefore, an outside diameter D4 of the insertion part 81 is set to be substantially equal to the inside diameter D1 of the housing space 37b of the auxiliary water feed port sleeve 42. As one example, the outside diameter D4 of the insertion part 81 is: inside diameter D4 = 6.15 mm. When fastening and fixing the check valve 75 to the connector 80, the external thread 82 engages with the internal thread (not illustrated) provided on the inner circumferential surface of the luer lock part 77 of the check valve 75.

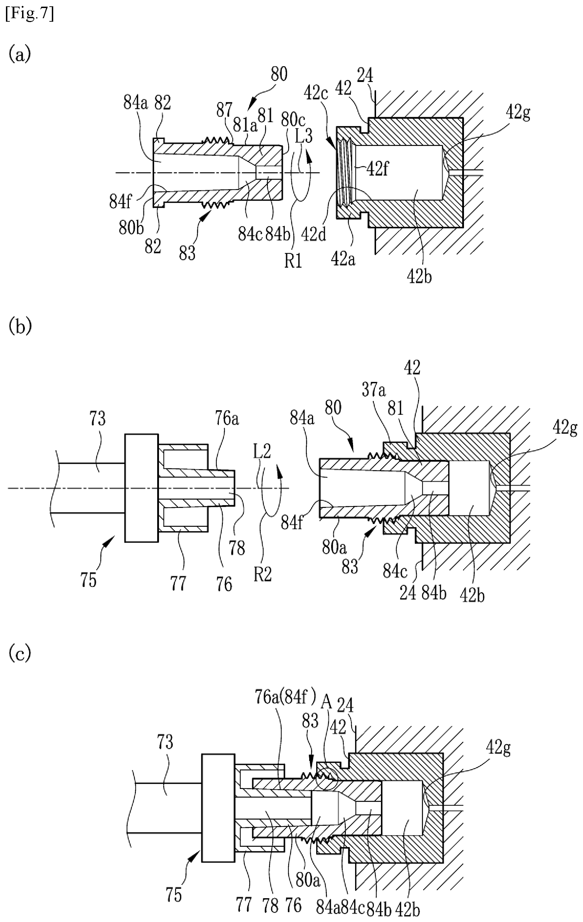

On the outer circumferential surface of the connector 80, the external thread 83 is provided within a predetermined range at a substantially central portion in the direction of the central axis (L3) of the connector 80. The external thread 83 engages with the internal thread 42c provided in the auxiliary water feed port sleeve 42. When the check valve 75 is fastened and fixed to the connector 80, the outer circumferential surface 76a of the luer tip 76 is brought into pressure contact with an inner circumferential surface 84f of a first flow passage 84a of the connector 80 to be described later (see Fig. 7(c)).

The connector 80 has a flow passage 84 that extends from the side of one end 80b toward the side of the other end 80c in the direction of the central axis (L3) of the connector 80. The flow passage 84 is a flow passage formed by connecting the first flow passage 84a, a second flow passage 84b, and a third flow passage 84c from the side of the one end 80b toward the side of the other end 80c in the direction of the central axis (L3) of the connector 80. Of these flow passages, the third flow passage 84c is a tapered flow passage that decreases in diameter from the first flow passage 84a toward the second flow passage 84b. A minimum value and a maximum value of the inside diameter of the first flow passage 84a are denoted by D5min and D5max, respectively; the inside diameter of the second flow passage 84b is denoted by D6; and a minimum value and a maximum value of the inside diameter of the third flow passage 84c are denoted by D7min and D7max, respectively. In this case, the minimum value D7min of the inside diameter of the third flow passage 84c is equal to the inside diameter D6 of the second flow passage 84b, and the maximum value D7max of the inside diameter of the third flow passage 84c is equal to the minimum value D5min of the inside diameter of the first flow passage 84a. The inside diameter D6 of the second flow passage 84b is smaller than the inside diameter D1 of the housing space 42b of the auxiliary water feed port sleeve 42, and is larger than an inside diameter D3 of the auxiliary water supply port 42g provided at the bottom part 42e of the housing space 42b.

Here, as one example, the minimum value D5min and the maximum value D5max of the inside diameter of the first flow passage 84a are: minimum value D5min = 3.75 mm, maximum value D5max = 4.33 mm. As one example, the inside diameter D6 of the second flow passage 84b is: inside diameter D6 = 1.5 mm. Further, as one example, the minimum value D7min and the maximum value D7max of the inside diameter of the third flow passage 84c are: minimum value D7min = 1.5 mm, maximum value D7max = 3.75 mm.

The flow passage 84 of the connector 80 here is formed by connecting the first flow passage 84a, the second flow passage 84b, and the third flow passage 84c from the side of the one end 80b toward the side of the other end 80c in the direction of the central axis (L3) of the connector 80. However, the flow passage 84 of the connector 80 may be any configuration that combines the first flow passage 84a extending from the side of the one end 80b toward the side of the other end 80c in the direction of the central axis (L3) of the connector 80 and the second flow passage 84b extending from the side of the other end 80c toward the side of the one end 80b in the direction of the central axis (L3) of the connector 80.

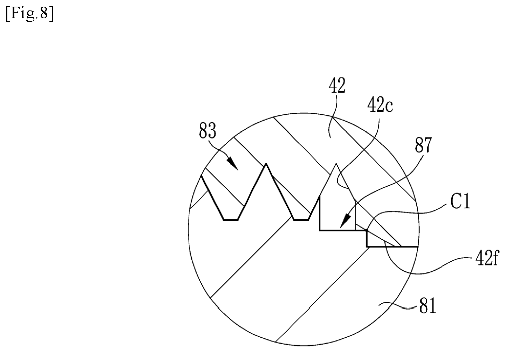

In the above-described connector 80, near an end portion of the external thread 83 on the side of the insertion part 81, a step 87 formed by the difference in outside diameter from the insertion part 81 is provided along the entire circumference. When the connector 80 is connected to the auxiliary water feed port sleeve 42, a ridge C1 of the step 87 (see Fig. 8) is brought into pressure contact with the tapered surface 42f provided in the auxiliary water feed port sleeve 42.

Next, a procedure of mounting the check valve 75 to the auxiliary water feed port sleeve 42 of the endoscope 20 using the connector 80 for an auxiliary water feed port sleeve in this embodiment will be described using Fig. 7.

As shown in Fig. 7(a), first, the insertion part 81 of the connector 80 is inserted into the housing space 42b provided in the auxiliary water feed port sleeve 42. In this state, the connector 80 is rotated, for example, in a direction R1 around the direction of the central axis (L3) of the connector 80 as the center. As the connector 80 is rotated in the direction R1, the external thread 83 of the connector 80 is engaged with the internal thread 42c of the auxiliary water feed port sleeve 42, so that the connector 80 is fastened and fixed to the auxiliary water feed port sleeve 42.

In the process of the connector 80 being fastened and fixed to the auxiliary water feed port sleeve 42, the ridge C1 of the step 87 provided in the outer circumferential surface of the connector 80 is brought into pressure contact with the tapered surface 42f provided between the inner circumferential surface 42d of the housing space 42b and the internal thread 42c provided in the auxiliary water feed port sleeve 42 (see Fig. 8).

The auxiliary water feed port sleeve 42 is made of a metal material, such as stainless steel, whereas the connector 80 is made of a synthetic resin material. Therefore, when the step 87 is brought into pressure-contact with the tapered surface 42f of the auxiliary water feed port sleeve 42 in the process of fastening and fixing the connector 80 to the auxiliary water feed port sleeve 42, the ridge C1 of the step 87 is brought into pressure contact with the tapered surface 42f of the auxiliary water feed port sleeve 42 while deforming (being crushed) due to the difference in hardness between the auxiliary water feed port sleeve 42 and the connector 80.

Next, the pump tube unit 70 is mounted onto the endoscope 20. The check valve 75 fixed to the water supply tube 73 of the pump tube unit 70 is connected to the connector 80 that has been connected and fixed to the auxiliary water feed port sleeve 42. First, the luer tip 76 of the check valve 75 is inserted into the first flow passage 84a of the connector 80. As shown in Fig. 7(b), the check valve 75 is rotated, for example, in a direction R2 around the direction of the central axis (L2) of the check valve 75 as the center. As the check valve 75 is rotated in the direction R2, the external thread 82 of the connector 80 is engaged with the internal thread of the luer lock part 77 of the check valve 75, so that the check valve 75 is fastened and fixed to the connector 80. As shown in Fig. 7(c), when the check valve 75 is fastened and fixed to the connector 80, an outer circumferential surface 76a of the luer tip 76 is brought into pressure contact with the inner circumferential surface 84f of the first flow passage 84a of the connector 80.

While here the check valve 75 fixed to the water supply tube 73 is fastened and fixed to the connector 80 after the connector 80 is fastened and fixed to the auxiliary water feed port sleeve 42, alternatively, the connector 80 may be fastened and fixed to the auxiliary water feed port sleeve 42 after the check valve 75 mounted on the one end portion of the water supply tube 73 is fastened and fixed to the connector 80. Otherwise, the check valve 75 and the connector 80 may be connected and fixed to each other in advance.

When the water supply device 50 is driven during an examination or a surgery using the endoscope 20, the liquid drawn into the pump tube 71 of the pump tube unit 70 is discharged to the water supply tube 73, while the liquid stored in the water supply tank 60 is drawn into the pump tube 71 through the filter-equipped water supply tube 72. While the water supply device 50 is operating, the discharge of the liquid drawn into the pump tube 71 to the water supply tube 73 and the intake of the liquid stored in the water supply tank 60 into the pump tube 71 are repeatedly performed. Thus, the liquid stored in the water supply tank 60 is intermittently delivered from the pump tube 71 to the water supply tube 73.

As described above, the water supply amount of the liquid in the pump tube unit 70 when the water supply device 50 is driven is 240 ml. In this case, the pressure of the liquid discharged from the pump tube 71 to the water supply tube 73 is, for example, 0.45 MPa.

The liquid discharged from the pump tube 71 to the water supply tube 73 flows from the flow passage 78 provided at the luer tip 76 of the check valve 75 to the first flow passage 84a of the connector 80. The liquid that has flowed into the first flow passage 84a of the connector 80 flows from the first flow passage 84a to the third flow passage 84c and the second flow passage 84b in this order.

As described above, when the check valve 75 is fastened and fixed to the connector 80, the outer circumferential surface 76a of the luer tip 76 of the check valve 75 is held in pressure contact with the inner circumferential surface 84f of the first flow passage 84a of the connector 80. Therefore, the liquid that has flowed from the flow passage 78 provided at the luer tip 76 of the check valve 75 into the first flow passage 84a of the connector 80 flows from the check valve to the flow passage 84 of the connector 80 without leaking between the outer circumferential surface 76a of the luer tip 76 of the check valve 75 and the inner circumferential surface 84f of the first flow passage 84a of the connector 80.

The liquid flowing through the flow passage 84 of the connector 80 flows from the second flow passage 84b toward the housing space 42b of the auxiliary water feed port sleeve 42. In this case, the speed of the liquid flowing from the first flow passage 84a to the second flow passage 84b increases as the liquid passes through the third flow passage 84c. Then, the speed of the liquid decreases when it flows from the second flow passage 84b into the housing space 42b of the auxiliary water feed port sleeve 42. Thus, the second flow passage 84b of the connector 80 functions as an orifice. The liquid that has flowed from the second flow passage 84b of the connector 80 into the housing space 42b of the auxiliary water feed port sleeve 42 is retained inside the housing space 42b, and after the inside of the housing space 42b is filled with the liquid, it flows from the water supply port 42g toward the auxiliary water supply channel SC.

As described above, the water supply port 42g, which continues to the auxiliary water supply channel SC, is provided at the bottom part 42e of the housing space 42b provided in the auxiliary water feed port sleeve 42. The inside diameter D3 of the water supply port 42g is smaller than the inside diameter D6 of the second flow passage 84b of the connector 80. Therefore, the liquid retained in the housing space 42b of the auxiliary water feed port sleeve 42 is pressed by the liquid that is newly supplied into the housing space 42b, and is thereby discharged from the water supply port 42g provided at the bottom part 42e of the housing space 42b into the auxiliary water supply channel SC.

Here, in the state where the insertion part 81 of the connector 80 has been fastened and fixed to the auxiliary water feed port sleeve 42, an outer circumferential surface 81a of the insertion part 81 of the connector 80 and the inner circumferential surface of the housing space 42b of the auxiliary water feed port sleeve 42 are in contact with each other. The ridge C1 of the step 87 of the connector 80 and a portion near the ridge C1 are held in a state of having been brought into pressure contact with the tapered surface 42f of the auxiliary water feed port sleeve 42 and thereby deformed. Thus, the portion near the ridge C1 of the step 87 of the connector 80 functions as a sealing material that is brought into pressure contact with the tapered surface 42f of the auxiliary water feed port sleeve 42. Since the outer circumferential surface of the insertion part 81 of the connector 80 and the inner circumferential surface of the housing space 42b of the auxiliary water feed port sleeve 42 are in contact with each other, liquid inflow is prevented between these surfaces. As a result, the occurrence of leakage from between the connector 80 and the auxiliary water feed port sleeve 42 is prevented even when the pressure of the liquid retained in the housing space 42b increases as the liquid is newly supplied from the connector 80 to the housing space 42b after the housing space 42b has been filled with the liquid.

When the examination or the surgery using the endoscope 20 ends, the connector 80 is removed from the auxiliary water feed port sleeve 42, and is disposed of along with the check valve 75 and the pump tube unit 70 to which the check valve 75 is connected.

Thus, a check valve that is used for another purpose can be connected to the endoscope 20 using the connector 80. Moreover, the connector 80 can function as a disposable product that can be disposed of along with the pump tube unit 70 after use.

Finally, results of performing the following Test (1) to Test (4) will be described:
(1) inspection for liquid leakage at the connection part
(2) measurement of the pressure of the liquid discharged from the auxiliary water feed port
(3) the discharge amount of the liquid discharged from the auxiliary water feed port of the endoscope
(4) measurement of a water retention time

Test (4) was performed at the same time as Test (3).

Hereinafter, the amount of the liquid discharged from the auxiliary water feed port 36 of the endoscope 20 will be referred to as a discharge amount. The amount of the liquid supplied toward the endoscope 20 as the water supply device 50 is driven will be referred to as a water supply amount. Here, the water retention time in Test (4) means the time from when the water supply device 50 is stopped until when discharge of the liquid from the auxiliary water feed port 36 of the endoscope 20 stops. Thus, a longer water retention time means poorer water drainage.

The aforementioned tests were performed using two different types of water supply devices 50. Hereinafter, one of the two types of water supply devices 50 will be referred to as a water supply device 50A, and the other one will be referred to as a water supply device 50B. The water supply amount that is actually supplied by each of the water supply device 50A and the water supply device 50B can be adjusted by, for example, changing (adjusting) the set level of the water supply amount provided in the device itself. The water supply amount of each of the water supply device 50A and the water supply device 50B becomes maximum when the set level is set to LEVEL9 and minimum when the set level is set to LEVEL1.

### <Selection of Set Level in Water Supply Device>

First, to perform the aforementioned Test (1) to Test (4), the set level of the water supply amount set in the water supply device 50A and the water supply device 50B was selected (determined). In selecting the set level, the following two different types of pump tube units were used:
Pump tube unit A: a pump tube unit dedicated to the water supply device 50A and having a pump tube with an inside diameter of 6.5 mm and an outside diameter of 9.6 mm
Pump tube unit B: a pump tube unit (the pump tube unit 70 having been shown in the embodiment) having a pump tube with an inside diameter of 3.6 mm and an outside diameter of 6.9 mm

As described above, the pump tube unit A is a dedicated pump tube unit for connecting to the auxiliary water feed port sleeve 42 of the endoscope 20 in a state of being attached to the water supply device 50A. While this is not illustrated, a dedicated check valve is fixed to the auxiliary water feed port sleeve 42 of the endoscope 20. A connection sleeve fixed at one end of the pump tube unit A is connected and fixed to the dedicated check valve.

The pump tube unit B is the pump tube unit 70 shown in the embodiment. The pump tube unit B is connected and fixed to the endoscope 20 by connecting and fixing the check valve 75, which is fixed at one end of the pump tube unit B, to the auxiliary water feed port sleeve 42 of the endoscope 20 through the connector 80 described in the embodiment.

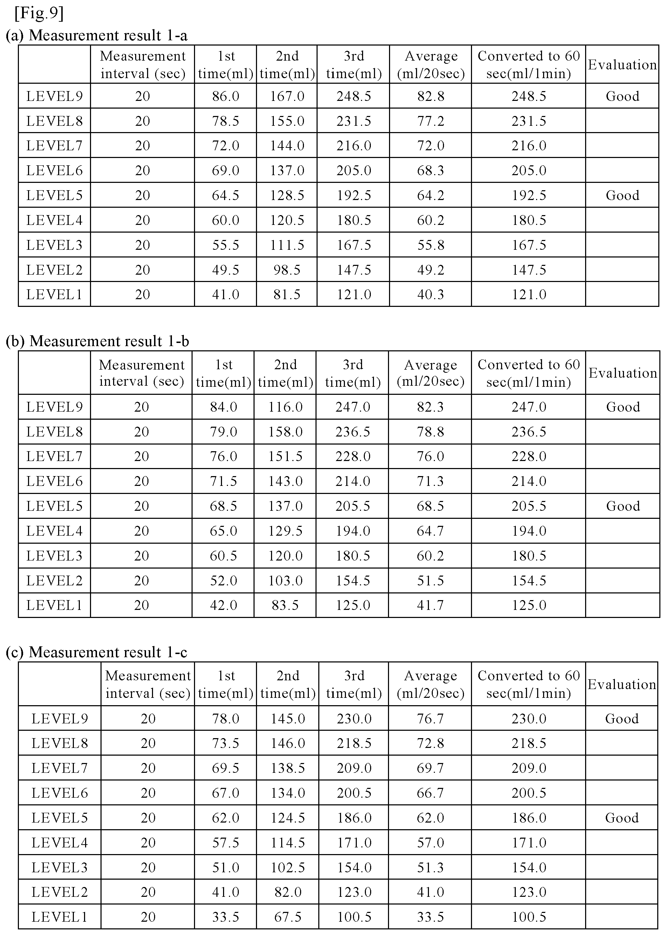

First, the discharge amount from the auxiliary water feed port 36 of the endoscope 20 was measured under the condition in which the water supply device 50A was driven with the pump tube unit A, attached to the water supply device 50A, connected to the endoscope 20. Fig. 9(a), Fig. 9(b), and Fig. 9(c) summarize the results of measuring the discharge amount discharged from the auxiliary water feed port 36 of the endoscope 20 (hereinafter referred to as measurement results 1-a, 1-b, and 1-c). The pump tube unit A attached to the water supply device 50A varied in accordance with the amount of water supplied as the water supply device 50A was driven; therefore, the discharge amount was measured three times at the same set level. The discharge amount from the auxiliary water feed port 36 of the endoscope 20 was measured at 20-second intervals, and the discharge amount per 60 seconds was calculated based on an average of the measured discharge amounts.

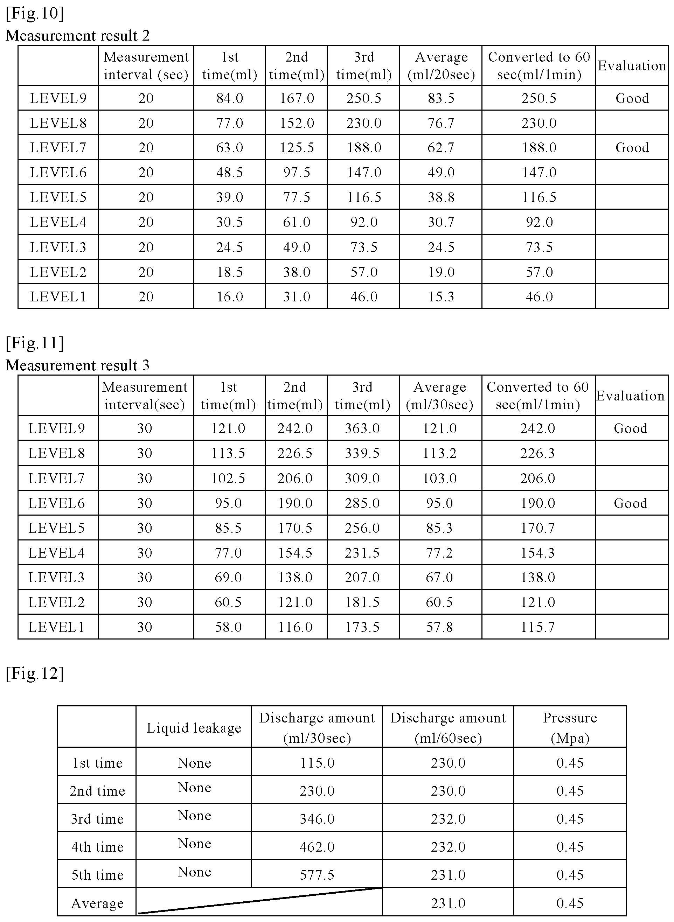

Next, the discharge amount from the auxiliary water feed port 36 of the endoscope 20 was measured while the water supply device 50A was driven with the pump tube unit B, attached to the water supply device 50A, connected to the endoscope 20. Fig. 10 summarizes the measurement result of the discharge amount from the auxiliary water feed port 36 of the endoscope 20 (hereinafter referred to as a measurement result 2). As in the previous case, the discharge amount from the auxiliary water feed port 36 of the endoscope 20 was measured at 20-second intervals, and the discharge amount per 60 seconds was calculated based on an average of the measured discharge amounts.

Finally, the discharge amount from the auxiliary water feed port 36 of the endoscope 20 was measured while the water supply device 50B was driven with the pump tube unit B, attached to the water supply device 50B, connected to the endoscope 20. Fig. 11 summarizes the measurement result of the discharge amount from the auxiliary water feed port 36 of the endoscope 20 (hereinafter referred to as a measurement result 3). The discharge amount from the auxiliary water feed port 36 of the endoscope 20 was measured at 30-second intervals, and the discharge amount per 60 seconds was calculated based on an average of the measured discharge amounts.

As shown in Fig. 9 and Fig. 10, the discharge amount of the liquid from the auxiliary water feed port 36 when the pump tube unit A was attached to the water supply device 50A, and the discharge amount of the liquid from the auxiliary water feed port 36 of the endoscope 20 when the pump tube unit B was attached to the water supply device 50A became the same discharge amount when becoming approximately 240 ml/min and about 190 ml/min. It was found that to set the set level of the water supply device 50A so as to achieve the aforementioned discharge amounts when using the pump tube unit A, the set level of the water supply device 50A should desirably be set to LEVEL9 or LEVELS. At the same time, it was found that to set the set level of the water supply device 50A so as to achieve the aforementioned discharge amounts when using the pump tube unit B, the set level of the water supply device 50A should desirably be set to LEVEL9 or LEVEL7.

The condition that the discharge amount of the liquid from the auxiliary water feed port 36 of the endoscope 20 is the same discharge amount is specified as a condition for the inspection. Therefore, when the pump tube unit B is used by being attached to the water supply device 50B, it is also preferable to adjust the set level of the water supply device 50B to achieve the same discharge amount as when the pump tube unit B is used by being attached to the water supply device 50A. As shown in Fig. 11, it was found that to set the set level of the water supply device 50B such that the discharge amount of the liquid from the auxiliary water feed port 36 of the endoscope 20 became about 240 ml/min or about 190 ml/min, the set level of the water supply device 50B should desirably be set to LEVEL9 or LEVEL6.

### <Test 1: Inspection for Liquid Leakage at Connection Part>

Next, an inspection test for liquid leakage at the connection part between the pump tube unit and the endoscope 20 was performed. First, inspection was performed for liquid leakage under the condition in which the pump tube unit B, attached to the water supply device 50B, was connected to the auxiliary water feed port sleeve 42 of the endoscope 20. The inspection for liquid leakage was performed with the set level of the water supply device 50B set to LEVEL9. The discharge amount from the auxiliary water feed port 36 of the endoscope 20 was measured at 30-second intervals, and the discharge amount per 60 seconds was calculated based on an average of the measured discharge amounts.

As shown in Fig. 12, the discharge amount of the liquid from the auxiliary water feed port 36 of the endoscope 20 was 231.0 ml/min. Inspection for liquid leakage performed in this case found no occurrence of liquid leakage at the connection part between the pump tube unit B and the endoscope 20, i.e., the connection part between the connector 80 and the check valve 75 and the connection part between the connector 80 and the auxiliary water feed port sleeve 42.

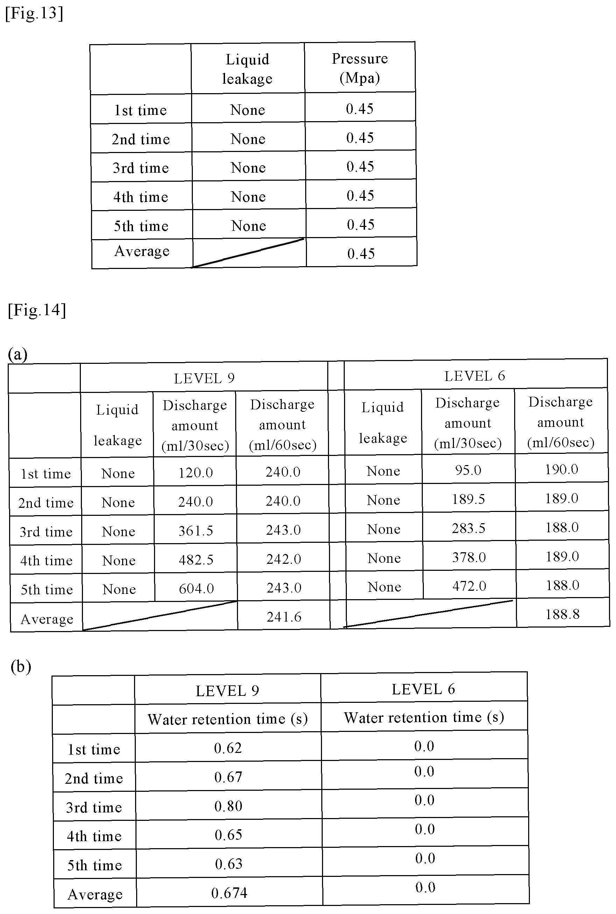

As comparative inspection, liquid leakage was examined when the pump tube unit A, attached to the water supply device 50A, was connected to the auxiliary water feed port sleeve 42 of the endoscope 20. As shown in Fig. 13, when the pump tube unit A, attached to the water supply device 50A, was connected to the auxiliary water feed port sleeve 42 of the endoscope 20, no liquid leakage was observed at the connection part between the pump tube unit A and the endoscope 20.

Thus, the results indicates that when the pump tube unit B attached to the water supply device 50B is connected to the auxiliary water feed port sleeve 42 of the endoscope 20 using the connector 80 in the embodiment, no liquid leakage occurs as when the pump tube unit A, attached to the water supply device 50A, is connected to the auxiliary water feed port sleeve 42 of the endoscope 20.

### <Test 2: Measurement of Pressure of Liquid Discharged from Auxiliary Water Feed Port>

A measurement test of the pressure of a liquid discharged from the auxiliary water feed port 36 was performed during the inspection for liquid leakage. As shown in Fig. 12, when the pump tube unit B attached to the water supply device 50B was connected to the auxiliary water feed port sleeve 42 of the endoscope 20, the pressure of the liquid discharged from the auxiliary water feed port 36 of the endoscope 20 was 0.45 MPa in all five measurements.

The pressure of the liquid was also measured when the pump tube unit A, attached to the water supply device 50A, was connected to the auxiliary water feed port sleeve 42 of the endoscope 20. As shown in Fig. 13, when the pump tube unit A attached to the water supply device 50A was connected to the auxiliary water feed port sleeve 42 of the endoscope 20, the pressure of the liquid discharged from the auxiliary water feed port 36 was 0.45 MPa in all five measurements.

Thus, it was found that connecting the pump tube unit B, attached to the water supply device 50B, to the auxiliary water feed port sleeve 42 of the endoscope 20 using the connector 80 in the present embodiment can ensure performance equivalent to that achieved when the pump tube unit A, attached to the water supply device 50A, is connected to the auxiliary water feed port sleeve 42 of the endoscope 20.

### <Test 3: Discharge Amount of Liquid Discharged from Auxiliary Water Feed Port of Endoscope and Test 4: Measurement of Water Retention Time>

Measurement of the discharge amount of a liquid discharged from the auxiliary water feed port 36 of the endoscope 20 and measurement of the water retention time were performed. In the measurement of the discharge amount of the liquid discharged from the auxiliary water feed port 36 of the endoscope 20 and the measurement of the water retention time, the following pump tube unit C was used in addition to the above-described pump tube units A and B:
Pump tube unit C: a pump tube unit having a pump tube with an inside diameter of 6.6 mm and an outside diameter of 9.7 mm.

The pump tube unit C is a pump tube unit having a pump tube with different inside diameter and outside diameter from the pump tube unit B. Like the pump tube unit B, the pump tube unit C is connected to the endoscope 20 as the check valve 75 fixed at one end of the pump tube unit C is engaged with and fixed to the auxiliary water feed port sleeve 42 of the endoscope 20 through the connector 80 having been described in the embodiment.

First, measurement was performed in the case where the pump tube unit B attached to the water supply device 50B was connected to the endoscope 20. As shown in Fig. 14(a), the average of the discharge amounts when the set level of the water supply device 50B was set to LEVEL9 was 241.6 ml/min. The average of the discharge amounts when the set level of the water supply device 50B was set to LEVEL6 was 188.8 ml/min. In the measurement of the discharge amount, no liquid leakage occurred at the connection part between the pump tube unit B and the endoscope 20.

As shown in Fig. 14(b), the average of the water retention times when the set level of the water supply device 50B was set to LEVEL9 was 0.674 s. The average of the water retention times when the set level of the water supply device 50B was set to LEVEL6 was 0.0 s.

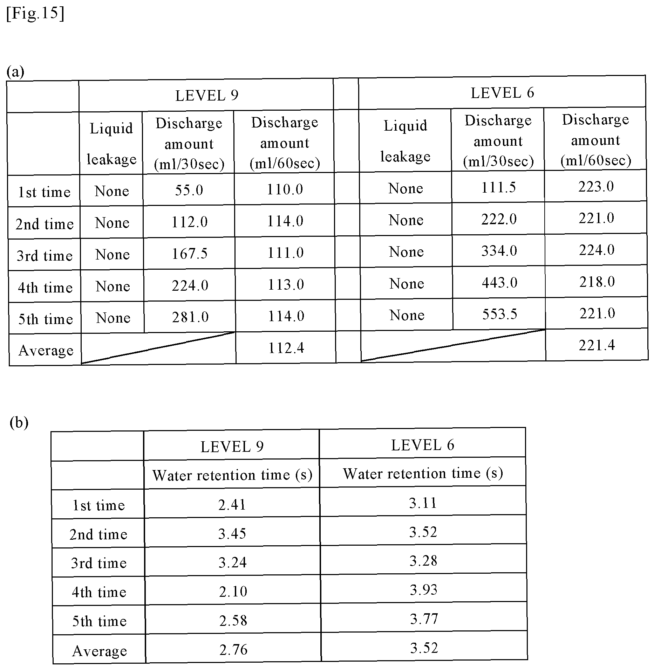

Next, measurement was performed in the case where the pump tube unit C having the pump tube 71 with an inside diameter of 6.6 mm and an outside diameter of 9.7 mm was attached to the water supply device 50B and connected to the endoscope 20. As shown in Fig. 15(a), the average of the discharge amounts when the set level of the water supply device 50B was set to LEVEL9 was 112.4 ml/min. The average of the discharge amounts when the set level of the water supply device 50B was set to LEVEL6 was 221.4 ml/min. In the measurement of the discharge amount, no liquid leakage occurred at the connection part between the pump tube unit C and the endoscope 20.

As shown in Fig. 15(b), the average of the water retention times when the set level of the water supply device 50B was set to LEVEL9 was 2.76 s. The average of the water retention times when the set level of the water supply device 50B was set to LEVEL6 was 3.52 s.

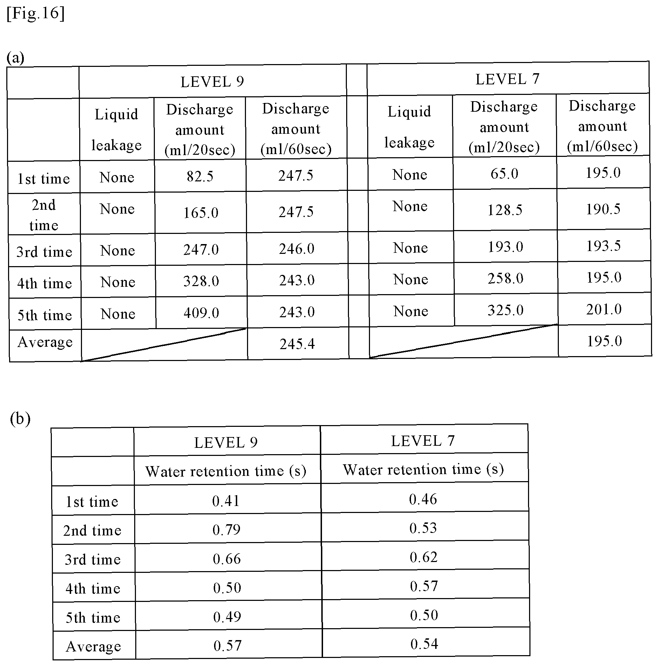

Next, measurement was performed in the case where the pump tube unit B was attached to the water supply device 50A and connected to the endoscope 20. As shown in Fig. 16(a), the average of the discharge amounts when the set level of the water supply device 50A was set to LEVEL9 was 245.4 ml/min. The average of the discharge amounts when the set level of the water supply device 50A was set to LEVEL7 was 195.0 ml/min. In the measurement of the discharge amount, no liquid leakage occurred at the connection part between the pump tube unit B and the endoscope 20.

As shown in Fig. 16(b), the average of the water retention times when the set level of the water supply device 50A was set to LEVEL9 was 0.57 s. The average of the water retention times when the set level of the water supply device 50A was set to LEVEL7 was 0.54 s.

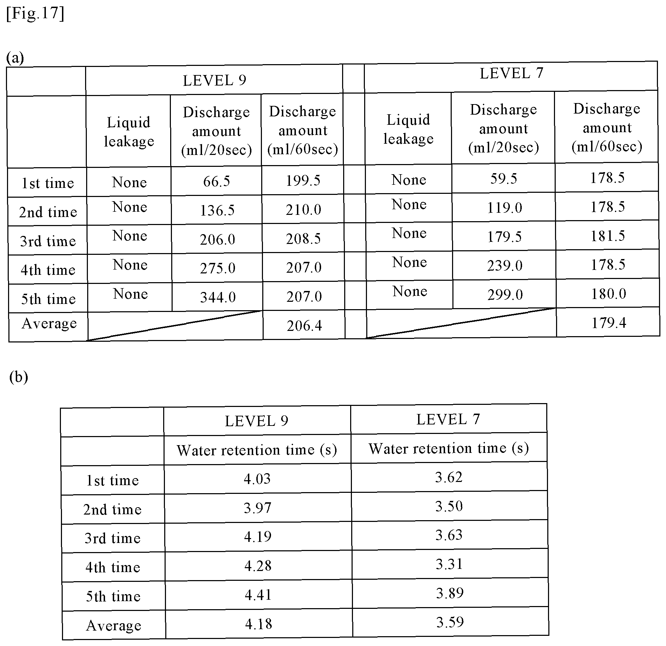

Next, measurement was performed in the case where the pump tube unit C was attached to the water supply device 50A and connected to the endoscope 20. As shown in Fig. 17(a), the average of the discharge amounts when the set level of the water supply device 50A was set to LEVEL9 was 206.4 ml/min. The average of the discharge amounts when the set level of the water supply device 50A was set to LEVEL7 was 179.4 ml/min. In the measurement of the discharge amount, no liquid leakage occurred at the connection part between the pump tube unit C and the endoscope 20.

As shown in Fig. 17(b), the average of the water retention times when the set level of the water supply device 50A was set to LEVEL9 was 4.18 s. The average of the water retention times when the set level of the water supply device 50A was set to LEVEL7 was 3.59 s.

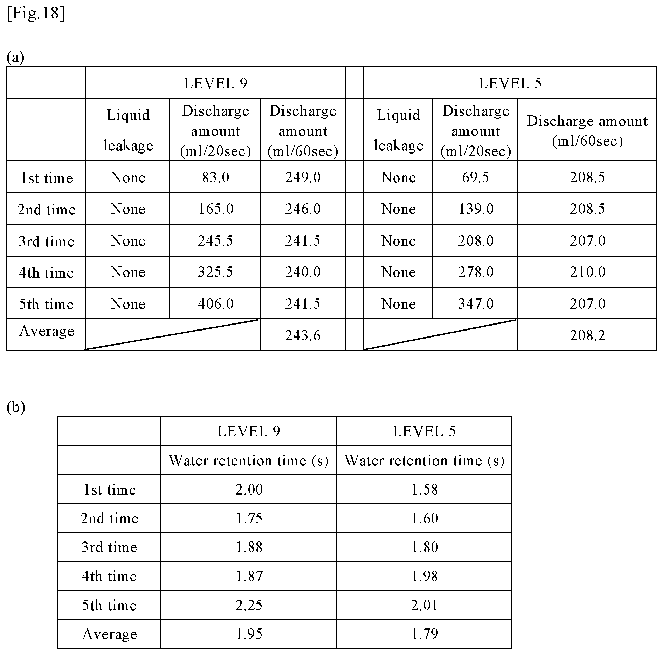

Finally, measurement was performed in the case where the pump tube unit A was attached to the water supply device 50A and connected to the endoscope 20. As shown in Fig. 18(a), the average of the discharge amounts when the set level of the water supply device 50A was set to LEVEL9 was 243.6 ml/min. The average of the discharge amounts when the set level of the water supply device 50A was set to LEVEL5 was 208.2 ml/min. In the measurement of the discharge amount, no liquid leakage occurred at the connection part between the pump tube unit A and the endoscope 20.

As shown in Fig. 18(b), the average of the water retention times when the set level of the water supply device 50A was set to LEVEL9 was 1.95 s. The average of the water retention times when the set level of the water supply device 50A was set to LEVEL5 was 1.79 s.

According to the measurement results of the water supply amount, it was found that when the pump tube unit B was attached to the water supply device 50B and connected to the endoscope 20, the discharge amount was more likely to meet the intended discharge amount than when the pump tube unit A was attached to the water supply device 50A and connected to the endoscope 20. As for the water retention time, it was also found that the water retention time when the pump tube unit B was attached to the water supply device 50B and connected to the endoscope 20 was shorter than that when the pump tube unit A was attached to the water supply device 50A and connected to the endoscope 20.

It was found that when the pump tube unit C was attached to either the water supply device 50A or the water supply device 50B and the set level was set to LEVEL9, there occurred a phenomenon in which the pump tube could not be appropriately squeezed while the water supply device was driven, so that water could not be supplied in the intended supply amount. On the other hand, when the set level was set to a level lower than LEVEL9, the water supply amount became close to the intended supply amount. Therefore, when using the pump tube unit C, the pump tube unit C should preferably be used at a set level lower than LEVEL9.

In conclusion, as a result of performing Test (1) to Test (4), it was found that attaching the pump tube unit B to the water supply device 50B or the water supply device 50A and connecting it to the endoscope 20 could secure performance equivalent to or higher than that when attaching the pump tube unit A to the water supply device 50A and connecting it to the endoscope 20. Moreover, it was found that in that case, connecting the pump tube unit B and the auxiliary water feed port sleeve 42 of the endoscope 20 to each other through the connector could inhibit the occurrence of liquid leakage and secure better water drainage. Thus, connecting the pump tube unit 70 that is not dedicated to the water supply device 50 to the endoscope 20 through the connector 80 can secure a level of performance equivalent to that when connecting the pump tube unit and the endoscope to each other using a dedicated part.

### <Summary of Advantages>

The connector 80 for an auxiliary water feed port sleeve of the present invention is used to connect the tube unit 70 installed in the water supply device 50 to the auxiliary water feed port sleeve 42 of the endoscope 20, characterized in that: the connector 80 for an auxiliary water feed port sleeve includes the tubular connector main body 80a made of a synthetic resin; the connector main body 80a has the insertion part 81 that is inserted into the housing space 42b provided in the auxiliary water feed port sleeve 42, the external thread 83 that is provided on the outer circumferential surface of the connector main body 80a and engages with the internal thread 42c provided in the housing space 42b of the auxiliary water feed port sleeve 42, and the step 87 that is provided between the insertion part 81 and the external thread 83 in the central axis direction of the connector main body 80a, along the entire circumference of the outer circumferential surface of the connector main body 80a; the auxiliary water feed port sleeve 42 has the tapered surface 42f between the housing space 42b and the internal thread 42c in the central axis direction of the auxiliary water feed port sleeve 42, the tapered surface 42f being reduced in diameter from the internal thread 42c toward the housing space 42b; and when the connector main body 80a is fastened and fixed to the auxiliary water feed port sleeve 42, the step 87 is held in pressure-contact with the tapered surface 42f.

Thus, the liquid that is supplied from the water supply tank 60 installed in the water supply device 50 as the water supply device 50 is driven is allowed to be reliably ejected from the auxiliary water feed port 36 through the auxiliary water supply channel SC of the endoscope 20, without leaking between the connector 80 for an auxiliary water feed port sleeve and the auxiliary water feed port sleeve 42 of the endoscope 20.

It is preferable that the connector main body 80a have the first flow passage 84a which is provided on the one end side in the central axis direction of the connector main body 80a and into which liquid from the pump tube unit 70 flows, and the second flow passage 84b which is provided on the other end side in the central axis direction of the connector main body 80a and from which the liquid flowing into the first flow passage 84a is discharged to the housing space 42b; that the inside diameter of the second flow passage 84b be smaller than the inside diameter of the first flow passage 84a; and that the inside diameter of the first flow passage 84a be smaller than the inside diameter of the housing space 42b.

Thus, the liquid is allowed to flow into the housing space 42b of the auxiliary water feed port sleeve 42, with the second flow passage 84b functioning as an orifice. In this case, the liquid having flowed into the housing space 42b of the auxiliary water feed port sleeve 42 has been reduced in pressure, and is thus prevented from exerting an excessive pressure load on the step 87 held in pressure-contact with the tapered surface 42f. As a result, the liquid can be reliably supplied to the auxiliary water supply channel SC of the endoscope 20 without leaking at the portion where the step 87 is held in pressure-contact with the tapered surface 42f.

It is preferable that the housing space 42b include, at the bottom part 42e of the housing space 42b, the water supply port 42g that communicates with the auxiliary water supply channel of the endoscope; that the inside diameter D3 of the water supply port 42g be smaller than the inside diameter D6 of the second flow passage 84b; and that the outside diameter D4 of the insertion part 81 be the same as the inside diameter D1 of the housing space 42b.

In this configuration, the liquid discharged by the pump tube unit 70 is retained in the housing space 42b of the auxiliary water feed port sleeve 42 through the connector, and is then discharged from the water supply port 42g to the auxiliary water supply channel SC under the pressure of the liquid that is newly discharged into the housing space 42b of the auxiliary water feed port sleeve 42. In this case, the liquid retained in the housing space 42b of the auxiliary water feed port sleeve 42 does not reach the portion where the step 87 is held in pressure-contact with the tapered surface 42f, as the insertion part 81 of the connector 80 and the housing space 42b of the auxiliary water feed port sleeve 42 come into contact with each other. Thus, leakage occurring between the connector 80 for an auxiliary water feed port sleeve and the auxiliary water feed port sleeve 42 of the endoscope 20 can be prevented.

It is preferable to connect the check valve 75, fixed at the one end of the pump tube unit 70, to the other end portion of the two end portions of the connector main body 80a in the central axis direction of the connector main body 80a, the other end portion being opposite from the one end portion connected to the auxiliary water feed port sleeve 42.

Thus, after the endoscope 20 is used, the connector 80 is disposed of along with the pump tube unit 70 and only the endoscope 20 is washed. Therefore, there is no need to use a check valve (or a check valve unit) dedicated to an endoscope, and instead a low-cost and disposable check valve that is used for another purpose can be used.

### Reference Signs List

10: endoscope system
20: endoscope
42: auxiliary water feed port sleeve
42f: tapered surface
50: water supply device
60: water supply tank
70: pump tube unit
75: check valve
80: connector for auxiliary water feed port sleeve
81: insertion part
83: external thread
84: flow passage
84a: first flow passage
84b: second flow passage
84c: third flow passage
87: step

## Claims

1. A connector for an auxiliary water feed port sleeve of an endoscope that is configured for use to connect a tube unit installed in a water supply device to an auxiliary water feed port sleeve of an endoscope, **characterized in that**:
the connector for an auxiliary water feed port sleeve of an endoscope comprises a tubular connector main body made of a synthetic resin;
the connector main body includes:
an insertion part that is inserted into a housing space provided in the auxiliary water feed port sleeve;
an external thread that is provided on an outer circumferential surface of the connector main body and engages with an internal thread provided in the housing space of the auxiliary water feed port sleeve; and
a step that is provided between the insertion part and the external thread in a central axis direction of the connector main body, along an entire circumference of the outer circumferential surface of the connector main body;
the auxiliary water feed port sleeve has a tapered surface between the housing space and the internal thread in a central axis direction of the auxiliary water feed port sleeve, the tapered surface being reduced in diameter from the internal thread toward the housing space; and
when the connector main body is fastened and fixed to the auxiliary water feed port sleeve, a ridge of the step provided along the entire circumference of the outer circumferential surface of the connector main body is held in pressure-contact with the tapered surface.

2. The connector for an auxiliary water feed port sleeve of an endoscope according to claim 1, wherein:
the connector main body includes:
a first flow passage which is provided on one end side in the central axis direction of the connector main body and into which a liquid from the tube unit flows; and
a second flow passage which is provided on the other end side in the central axis direction of the connector main body and from which the liquid flowing into the first flow passage is discharged to the housing space;
an inside diameter of the second flow passage is smaller than an inside diameter of the first flow passage; and
the inside diameter of the first flow passage is smaller than an inside diameter of the housing space.

3. The connector for an auxiliary water feed port sleeve of an endoscope according to claim 2, wherein:
the housing space includes, at a bottom part of the housing space, a water supply port that continues to an auxiliary water supply channel of the endoscope;
an inside diameter of the water supply port is smaller than the inside diameter of the second flow passage; and
an outside diameter of the insertion part is equal to the inside diameter of the housing space.

4. The connector for an auxiliary water feed port sleeve of an endoscope according to claim 3, wherein
a check valve fixed at one end of the tube unit is connected to the other end portion of the two end portions of the connector main body in the central axis direction of the connector main body, the other end portion being opposite from one end portion connected to the auxiliary water feed port sleeve.
